# EUROPEAN PATENT SPECIFICATION

(11) **EP 1 347 723 B1**
(45) Date of publication and mention of the grant of the patent: **21.02.2007**
(21) Application number: 01272615.4
(22) Date of filing: 21.12.2001
(51) Int. Cl.: A61F 5/453, B29C 41/14, B29C 55/22

(54) **AN EXTERNAL URINARY CATHETER DEVICE AND A METHOD FOR PRODUCTION THEREOF**
EXTERNE HARNKATHETERVORRICHTUNG UND VERFAHREN ZU IHRER HERSTELLUNG
CATHETER URINAIRE EXTERNE ET PROCEDE DE FABRICATION

(30) Priority: 03.01.2001 DK 200100008; 10.05.2001 DK 200100735
(43) Date of publication of application: 01.10.2003
(73) Proprietor: COLOPLAST A/S, 3050 Humlebaek (DK)
(72) Inventor: GULDFELDT, Signe, Uhre, DK-3400 Hillerod (DK); NIELSEN, Henrik, Lindenskov, DK-2765 Smorum (DK); TANGHOJ, Allan, DK-2980 Kokkedal (DK)
(74) Representative: Laudrup, Peter
(86) International application number: PCT/DK2001/000862
(87) International publication number: WO 2002/053070

(56) References cited:
- EP-A- 0 068 712
- EP-A- 0 706 355
- US-A- 4 656 675
- US-A- 5 263 947
- US-A- 5 401 264
- US-A- 5 554 141
- US-A- 5 779 964

## Description

### Introduction

The present invention relates to an external urinary catheter device for the relief of male urinary incontinence, comprising a contact member adapted for arrangement, in a position of use, in engagement with at least an extreme portion of a penis; an opening formed in a distal end section of the contact member to be positioned substantially opposite the urethral orifice in the position of use, and a discharge conduit connected with the opening to provide a sealed urine flow passage extending in a substantial axial direction towards the exterior of the catheter device.

The invention also pertains to a method and processes for production of an external urinary catheter device for the relief of male urinary incontinence as well as use of catheters of this kind. A urinary catheter and a method for production thereof as defined in the preamble of claims 1 and 21, respectively, is disclosed in US-A-4 656 675.

### Background art

For the relief of male urinary incontinence external catheters are generally used in the form of condom-like tubular sheaths to be placed externally on penis and having a discharge spout, which via a hose is connected with a urine collection bag. Such external catheters are known in numerous designs and in many cases serve as a satisfactory solution of male incontinence problems. However, the complete envelopment of penis may give rise to trouble, partly because the application, which is effected by unrolling the catheter requires a certain length of penis, partly in use due to the fact that the envelopment of the full length of penis with the catheter, which is generally fastened adhesively either by means of a separate adhesive strip or by means of an internal adhesive layer, involves strain of the skin of penis. Furthermore, the constant humid environment from the delivered urine may cause skin problems, such as allergy and maceration and even ulceration.

In recent years, various suggestions have been presented in the prior art concerning the use in a urinary catheter of an inner member or a contact member, which in a position of use is positioned between the surface of the corona and the foreskin of the penis.

Thus, from GB-A-2 075 847 a device is known, which comprises a relatively short funnel-like external catheter device to be placed directly against the corona of the penis and to be kept in place under the foreskin. Around a discharge conduit of the device an external sheathing body is fastened in an inverted position to be brought into engagement with the external side of the foreskin after application of the external catheter device to maintain the device in place by means of the sandwich position of the foreskin between the contact member and the external sheathing body.

Another design is disclosed in GB-A-2 126 483, wherein a more safe retainment of the device in the position of use is implemented by use of a tubular urine ducting device, e.g. of silicone rubber, arranged outside the corona in extension thereof, so that a rather severe extension of the foreskin is required to keep the tubular device in place by arrangement of an adhesive strip or strap member on the external side of the foreskin. Evidently, the rather severe extension of the foreskin required to keep the tubular device in place causes considerable discomfort in use.

In EP-B-0 706 355 and EP-B-0 706 356 external urinary catheters are disclosed, in which a safe retainment in the position of use is obtained with considerably less discomfort to the user by means of a short inner contact member designed with improved form stability in its distal end part. Aiming at keeping the contact member safely in place under the foreskin the catheter is provided with a separate substantially bowl-shaped outer holder member displaceably surrounding a discharge conduit of the catheter device. The outer holder member is movable between an application position and a position of use.

Urinary catheters are generally connected via a hose to a urine collection bag. The wearer of the urinary catheter suitably places the urine collection bag in a position lower than the position of the urinary catheter to ensure that the urine effectively is drained off by gravity. To allow free movement of the wearer of the urinary catheter the urine collection bag is generally positioned at a part of the leg. Generally, it is preferred to position the urine collection bag at the shin to drain off urine in a standing as well as a sitting position. Furthermore, this position of the urine collection bag provides for the possibility of hiding it behind a trouser leg.

When using the prior art catheters mentioned above the problem arise, following a discharge of urine, that the unbroken column of urine in the hose give rise to a considerable built-up of sub-pressure at the surface of the corona. The wearer of the urinary catheter is subjected to discomfort, especially on extended exposure to the sub-pressure. Suction marks have been observed on the corona due the exposure to sub-pressure. In severe cases, oedema and subsequent infection has been developed.

It has been suggested to overcome this problem by incorporating a vent or a valve in the urine collection bag or the lower part of the hose allowing the surrounding air to communicate with the interior of the catheter-hose-bag environment. Reference is made to e.g. US 3,800,857, US 3,835,857, and US 5,622,183. However, the position of a vent or valve in the bag or the lower part of the hose entails the potential risk that urine, when the bag is full or about to be full, will escape the urine collection bag.

Another suggestion is disclosed in US 5,897,540 wherein a slit-shaped opening is provided in a condom-like device for drainage of uncontrolled urine release. The opening is provided for ventilation of the space entrapped between the device and the penis and is placed in the position of use at the main body of the penis well behind the corona. The opening is not provided with a filter or similar means. A further suggestion is disclosed in US 4,656,675, which describes a condom-like device for draining off urine. The device is provided with a filter covering a hole positioned at a point close to the source of urine. The filter is capable of passing air but impervious to liquid at relative low pressures. A major drawback of this suggestion is that the filter is placed at an exposed position. The wearer of the device must be careful since the filter easily can be ripped off by abrasion or accident and the urine will tend to be conveyed through the hole in the device and not as intended through the hose.

It is the object of the present invention to avoid all or some of the above discussed problems and drawbacks of the prior art urinary catheters. Especially, it is an object of the present invention to provide a urinary catheter device having an enhanced reliability and durability due to reduced risk of deterioration of a filter means.

### Description of the invention

According to one aspect of the invention, an external urinary catheter device for the relief of male urinary incontinence is provided, which comprises
a contact member adapted for arrangement, in a position of use, in engagement with at least an extreme portion of a penis,
an opening formed in a distal end section of the contact member to be positioned substantially opposite the urethral orifice in the position of use,
a discharge conduit connected with the opening to provide a sealed urine flow passage extending in a substantial axial direction towards the exterior of the catheter device,
an aperture provided in a distal part of the contact member or in the discharge conduit,
a membrane attached to the surface surrounding said aperture and capable of selectively passing gases but retaining urine,
an external protective shield arranged for at least partly covering the membrane, and
at least one vent provided for allowing gases to be in substantially unhindered contact with the external surface of the membrane.

The contact member and optionally also further components of the catheter device according to the invention, such as the discharge conduit and the shield, may be made from any material which is suitable for engagement with the sensitive skin of the corona and the foreskin. Suitable materials include thermoplastic elastomeric materials like styrene-butadiene-styrene (SBS), styrene-isoprene-styrene (SIS), styrene-ethylene/butadiene-styrene (SEBS), styrene-ethylene/propylene-styrene (SEPS), polyethers, polyesters, polyamides, polyurethane or any combination thereof; thermoplastic materials such as plasticized polyvinylchloride (PVC), and polyethylene; or curable elastomeric materials like nitril rubber, chloroprene rubber, natural rubber latex, silicone rubber, liquid silicone, polyisoprene or polyurethane. Preferred materials are SEBS and silicone rubber.

The membrane in the device according to the invention allows gas to pass, whereby it is ensured that the amount of urine discharged from a wearer may be drained to a urine collection bag via a hose without leaving a substantial amount of urine in the hose giving rise to a sub-pressure at the surface of the corona. The term gas as used in the current context means normally ambient air (a gas mixture of predominately nitrogen, oxygen, and water vapour). However, other gases or compositions of gases may come into play. Following a urine discharge the column of urine in the hose will gradually sink to an equilibrium level such that the pressure in the hose above the urine surface essentially corresponds to the pressure of the surrounding environment. A minor column of urine may be present due to a capillary effect and resistance in the membrane, however without entailing a substantial pressure effect on the surface of the corona.

The aperture provided in the distal part of the contact member or the discharge conduit may have any suitable dimension. The longest diagonal of the aperture may be larger than the diameter of the discharge conduit. However, to avoid a weakening of the discharge conduit, it is preferred that the aperture has a longest diameter less than the diameter of the discharge conduit, or the aperture may extend throughout the circumference of the discharge conduit. A suitable longest diagonal of the aperture is about 0,25 to 0,75 times the inner diameter of the discharge conduit. Preferably the circumference of the aperture is a circle, however other geometrical shapes might be suitable, such as ovals or polygons. Further suitable shapes are constituted by organic shapes. If desired, more than one aperture may be present in the device. The aperture is preferably positioned at a part of the discharge conduit close to the urethral orifice or the distal part of the contact member to obtain sufficient ventilation. Generally, it is to be avoided to arrange the aperture in a part of the discharge conduit intended to engage with a connector linking the device of the invention with a hose member, to prevent the risk of insufficient gas communication between the inner surface of the membrane and the interior of the discharge conduit. A preferred position of the aperture is in the proximal part of the discharge conduit close to the urethral orifice in a position of use.

The membrane is fastened to the surface surrounding the aperture, the membrane being capable of selectively passing gases but retaining urine. During discharge of urine from the urethral orifice the membrane effectively prevent urine from penetration and thus secure a sealed urine flow passage. Following the discharge of urine gas from the surrounding environment is penetrating the pores of the membrane and consequently, the column of urine in the hose is levelled. The membrane suitably maintains the desired selectivity at the relatively low pressures normally expected during use of the catheter device according to the invention. Normally, the water entry pressure of the membrane should be at least 100 mbar above ambient pressure.

The membrane is generally a thin, hydrophobic, porous sheet having the ability of allowing gas, i.e. air, to penetrate but not urine, which is retained. A wide range of materials may be used for the membrane. Preferred materials are generally polymers with a low surface tension to water. Examples of suitable polymers are polyethylene, polypropylene, polyurethane, and polytetrafluorethylene (PTFE). Membranes of PTFE are commercially available under various trademarks, e.g. Goretex^{®}. A preferred membrane of polyethylene is commercially available under the trademark Tyvek^{®}.

While the membrane in one aspect of the invention is designed to retain urine at least at the relatively low pressures expected during normal use, it may be desirable, in another aspect of the invention, to use a membrane which at pressures above the level normally expected during use, is adapted to allow the passage of urine to avoid backing up of urine against the penis, creating a risk for reflux, or for ballooning of the foreskin. As an example, a sudden rise in pressure is obtained if the hose is kinked. In accordance with a second aspect of the invention the membrane may be adapted to allow the passage of urine at pressures over 80 mbar above ambient pressure, preferably over 100 mbar above ambient pressure.

The fastening of the membrane to the surface surrounding the aperture is normally conducted in order to secure a watertight attachment of the membrane around the aperture. Any suitable method of fastening may be applied, including adhering, riveting, partly dissolution by solvents, mechanical stress, or welding. Various suitable welding methods exist e.g. thermal, high frequency, ultrasonic, induction, or laser welding. In one aspect of the invention, an adhesive is used for adhering the membrane to the surface surrounding the aperture, since this method provides a simple and cost-effective way to obtain an effective attachment of the membrane to the surface surrounding the aperture. In another aspect of the invention, a rivet is used to fasten the membrane to the surface surrounding the aperture, whereby the membrane is fixed between the male and the female parts of the rivet to providing an effective mechanical attachment of the membrane to the surface surrounding the aperture.

In still another aspect of the invention, fastening of the membrane to the surface of the aperture is obtained by melting the surface surrounding the aperture to an opposite surface of the membrane by welding or exposure to solvents. It is, in general, preferred to use welding, which includes applying an amount of heat sufficient to melt a part of the plastic material, allowing penetration of the melted plastic material into the pores of the membrane and subsequent allowing solidification of the plastic material. Thus, a simple and reliable way to attach the membrane to the surface around the aperture is obtained. A method especially suitably for attaching the membrane to the surface surrounding the aperture is ultrasonic or thermal welding.

In some embodiments of the invention it is advantageous to depress or sink an area surrounding the aperture and arrange the membrane in this depression. In such embodiments a space above the external surface of the membrane is generally obtained which allow sufficient ventilation of gases to and from the membrane surface.

In a preferred embodiment of the invention the contact member is integrally connected with the discharge conduit via the opening formed in the distal end section of the contact member. This embodiment makes it possible in a single component of the catheter device to integrate several functions, thus, increasing the industrial processability and securing a sealed urinary flow passage. In some areas of application, it may be useful to connect the discharge conduit to the opening formed in the distal end section of the contact member through a stretchable link to maintain the intimate engagement of the contact member with the penis in a position of use even under tensile load conditions acting on the discharge conduit. The stretchable link may for instance be a bellows-shaped member.

In a preferred embodiment of an external urinary catheter device according to the invention the contact member adapted for arrangement in engagement with a penis may constitute an inner member arrangeable in a position of use between the foreskin and the corona of the penis, whereby the device may further include an outer holder member adapted to be positioned against the external surface of the foreskin to provide a sealed contact.

In case the contact member is an inner member arrangeable in a position of use between the foreskin and the corona of a penis it may comprise a shoulder-like ledge at a substantially plane abutment face of the inner member around and substantially perpendicular to the discharge conduit to retain the inner member in contact with the corona solely by means of the foreskin contacting the outer side of the contact member. An inner member of this kind is disclosed in EP 706 355 B1, the entire disclosure thereof being incorporated herein by reference.

Another suitable inner member which is arrangeable between the foreskin and the corona of a penis or solely on the corona of a penis e.g. in the absence of foreskin in case of circumcising, comprises a plurality of leaves connected to a housing. The leaves are comprised of a film having an adhesive layer on their interior surface thereof to secure an attachment of the device to the corona of a penis. Preferably, the film as well as the adhesive layer is vapour permeable to increase the comfort of the wearer. An inner member of this kind is disclosed in US 5,263,947.

For fastening the inner member in a position of use, it may be advantageous to provide the catheter device according to the invention with an outer holder member. The outer holder member is preferably formed in a distal end section with an opening for the discharge conduit and arrangeable in said position of use, in a substantially fixed axial position with respect to the discharge conduit for circumferential engagement with the external side of the foreskin. An outer holder member of this kind is disclosed in EP 760 356 B1.

The outer holder member may be made of any suitable soft elastic, semi-rigid or rigid material such as the plastic materials mentioned above for the contact member or, alternatively, it may be made in the form of a web of textile material like gauze, hydrocolloid skin adhesive covered with a film, or non-textile material. For improved engagement with the external side of the foreskin the inner engagement surface of the outer holder member may be coated with a layer of a skin-friendly adhesive, e.g. a hydrocolloid skin adhesive. Such an adhesive layer may be dimensioned with controlled adhesive properties and/or be applied in various patterns as known in the art to control the adhesive force. By use of an adhesive layer the length of the outer holder member in the position of use may be reduced to cover an even shorter part of the foreskin in order to increase comfort in connection with wear or removal of the catheter device.

The inner member may be permanently connected with a surrounding outer holder member having an opening for the discharge conduit by connection means. Suitably, said connection means is designed also to comprise the shield for the catheter according to the invention. Thus, the shield is preferably an extended part of the distal end section of the outer holder member. The connection means serving the dual purpose of permanently connecting the contact member with the outer holding member and shielding the membrane is in one aspect of the invention an essentially tubular member arranged around and having walls substantially parallel to the axis of the discharge conduit and integrally connected to the opening of he outer holder member.

The preferred materials for the individual parts of the device usually provides for an inherent adherence between the inner face of the tubular member and the outer face of the discharge conduit, if the individual parts of the catheter are properly designed. However, the arrangement of the membrane between the tubular member and the discharge conduit may jeopardise the inherent adherence because the materials for the membrane usually do not exhibit the adherence properties. Therefore, in another aspect of the invention, the inner member is fixed in at least an axial direction relative to the outer holder member by fastening means. As used in the present description and claims, the term fixed is to be understood as an adherence between the tubular member and the discharge conduit, which may allow for some flexibility due to softness and/or shape of the materials used.

In an embodiment of the invention, the outer holder member is fixed in the axial direction by providing the discharge conduit with a ledge outside the outer edge of the tubular member. The ledge secures that the outer holder member connected to the tubular member is not moved substantially in the axial direction, when the outer holder member in use is pulled back and forward. However, a minor free fit can be allowed if considered expedient, e.g. for customizing the catheter to the anatomy of the individual user

The ledge may extend over the entire circumference of the discharge conduit or a part thereof. In another embodiment the inner face of the tubular member and the outer face of the discharge conduit may be provided with corresponding recesses and ledges to secure a locked position of the parts of the device at least in the axial direction. If the tubular member is of a resilient material the fastening may be provided by one or more ledges in the inner face of the tubular member and/or the outer face of the discharge conduit without corresponding recesses in the counterpart.

The outer holder member and the inner member may be fixed in an axial as well as a rotational direction by fastening means. Suitably, the fixation is provided by fastening a tubular member integrally connected with the outer holder member to the discharge conduit, which is connected to the inner member. The fastening means may be of any suitable type. Especially, the fastening means may be conducted as described for the fastening of the membrane to the surface surrounding the aperture. In some practical designs of the device the tubular member may be attached to the discharge conduit by thermal or ultrasonic welding. The welding may be conducted in an area, in which a part of the membrane is sandwiched between the tubular member and the discharge conduit to provide for the dual function of fastening the membrane to the surface surrounding the aperture and fixing the tubular member to the discharge conduit. The welding may be a point welding. In this case, preferably more than one welding is provided to ensure that the stress delivered to the outer holder member during application or carrying of the device is more equally distributed. Preferably 2, 3 or 4-point weldings are provided.

In still another embodiment of the invention, the fastening of the outer member relative to the inner member in at least the axial direction can be provided by mechanical means. As an example, the area of the tubular member above the transition for the invertible outer holder member may be provided with a collar band or a resilient member, which is able to tighten the tubular member to the discharge conduit. The fastening may also be provided by a membrane device. The membrane device may be installed in an aperture through the tubular member as well as the discharge conduit. The membrane device has the ability to retain the tubular member and the discharge conduit in position relative to each other and to provide for the compulsory membrane. The membrane device may be locked in position in the aperture by any suitable fastening means. As an example, the membrane device may be formed as a rivet or the device may be adhesively bonded to the surface surrounding the aperture.

The contact member adapted to be in engagement with the penis, may in an aspect of the invention be adapted to be positioned on the external face of a penis to provide a sealed contact. To ensure an effective sealing between the contact member and the penis the contact member is suitably of a length covering the extreme portion, e.g. the foreskin or the corona, as well as the main body of the penis. An aperture can suitably be provided in a proximal part of the discharge conduit and covered with a membrane. A shield may be provided over the membrane as a separate or integral tubular member with an internal diameter substantially corresponding to or slightly smaller or greater than the external diameter of the discharge conduit to fix the membrane. A stiffening member may be arranged inside the discharge conduit to provide a suitable abutment for the tubular member. The tubular member can be provided with one or more flaps for facilitating the removal of the device

The urinary catheter of the invention comprises at least one vent, which is arranged for allowing the gases, e.g. air, to be in substantially unhindered contact with the external surface of the membrane. The vent may be provided in any suitable way to ensure that the gases virtually unhindered can be conveyed from or to at least a part of the surface of the membrane. Thus, vents may be provided in the shield, the discharge conduit or both. Alternatively, the vent may be one or more hollow fibres or the like provided between the inner face of the shield and the outer surface of the discharge conduit to allow gas communication between the external face of the membrane and the surrounding environment.

In an aspect of the invention the vent is a single aperture provided in the shield. The aperture in the shield covers preferably the entire active membrane area to allow sufficient ventilation, however, especially in embodiments where the inner face of the shield is not in direct contact with the membrane, it may be desirable to provide the catheter according to the invention with an aperture smaller than the active membrane area to obtain an extended protection of the membrane.

In another aspect of the invention, the shield is provided with a plurality of apertures for ventilation. This aspect is of particular interest when a space above the external surface of the membrane is provided, because the entire membrane area may be active. A space above the external membrane may be provided e.g. by fasting the membrane with a rivet or by attaching the membrane in a depression of the external surface of the discharge conduit. A plurality of relatively small apertures relative to a single larger aperture is regarded to be more effective for protecting the membrane since sharp or pointed devices to a larger extent is prevented from coming into intimate contact with the membrane from the outside of the catheter device.

A vent passage may also be provided using one or more tracks for channelling the gases to or from the external surface of the membrane to the surrounding environment. In particular, one or more track(s) may be provided in the inner face of the shield to establish a vent passage from the external surface of the membrane to the exterior of the catheter device.

In one aspect of the invention a plurality of tracks substantially parallel to the axis of the discharge conduit are formed in the inner face of the shield in a part or the entire circumference thereof establishing a vent passage from the external surface of the membrane to the exterior of the catheter device. The aspect of using tracks in the entire circumference of the inner surface of the shield could be advantageous from a production point of view, as the assembling of the catheter is thereby made independent of the relative position of the shield and the membrane due to the rotational symmetry of the shield.

The depression receiving the membrane may be provided with one or more depressed track(s) extending in a distal direction beyond the edge of the shield, said track(s) establishing a vent passage from the external surface of the membrane to the exterior of the catheter device. This alternative is attractive from a production point of view since the mould in which the device is formed, may be designed to provide an impression in the catheter part, said impression having the dual purpose of receiving the membrane and establishing an effective ventilation through the track(s). Moreover, the shield may have a tubular rotational symmetrical shape making it possible to assemble the catheter without observing the relative position of the shield and the membrane.

To obtain a large cross-section of the vent(s) one or more track(s) may be provided in the discharge conduit as well as the inner face of the shield, said tracks in common providing one or more channels for ventilation.

The track(s) provided in the discharge conduit and/or the inner face of the shield may have any suitable shape. Normally, the tracks are parallel to the axis of the discharge conduit to obtain the shortest channel. Other shapes may be considered to obtain specific properties of the catheter. As an example, the track may be provided with one or more bends to trap dust and to avoid penetration of pointed devices. As another example, tracks on the discharge conduit may be thread-like, whereby the gas is conducted around the axis of the discharge conduit.

The invention also relates to a method for production of an external urinary catheter device for the relief of male urinary incontinence, which comprises the steps of
providing a catheter part comprising a contact member which is adapted to be engaged with a penis; an opening formed in a distal end section of the contact member and positioned substantially opposite the urethral orifice in the position of use; and a discharge conduit connected with the opening to provide a sealed urine flow passage extending in a substantial axial direction towards the exterior of the catheter device,
providing an aperture in a distal part of the contact member or in the discharge conduit,
attaching a membrane to the surface surrounding said aperture, the membrane being capable of selectively passing gases but retaining urine,
providing a protective shield for at least partly covering the membrane, and
arranging at least one vent for allowing gases to be in substantially unhindered contact with the external surface of the membrane.

The catheter part comprising the contact member, opening and discharge conduit may be assembled of individual members or be produced in one piece. It is preferred to produce the catheter part in one piece due to the process economy by integrally connecting the contact member to the discharge conduit via the opening formed in the distal end section of he contact member. The catheter part may suitably be produced by plastic injection moulding using the polymer materials previously mentioned.

The aperture may be provided in any suitably way ensuring that gases substantially unhindered can communicate with the internal face of the membrane. In case the catheter part is produced in one piece by plastic injection moulding the mould is suitably adapted to provide the aperture during manufacture of the catheter part.

The membrane fastened to the surface surrounding the aperture is suitably fastened such that it is secured that a waterproof attachment is obtained. The fastening procedure may be any suitable method securing a waterproof attachment, including adhering, riveting, partly dissolution by solvents, and welding. A preferred method of attachment comprise melting the surface surrounding the aperture to an opposite surface of the membrane. A preferred method of fastening the membrane to the surface surrounding the aperture is conducted by welding. Various welding methods are suitable, including ultrasonic, high frequency, induction, laser, and thermal welding. It is in general preferred to conduct the fastening by ultrasonic or thermal welding.

In an aspect of the invention, the above method comprises the further step of providing the catheter part with an outer holder member for fastening the contact member in the position of use, wherein the outer holder member is formed in a distal end section with an opening for said discharge conduit and arrangeable in a position of use, in a substantially fixed axial position with respect to the discharge conduit for circumferential engagement with the external side of the foreskin.

In one preferred form of the method a dip forming process is used for manufacture of the external urinary catheter device for the relief of male urinary incontinence, comprising the steps of
a) providing a liquid polymer solution or emulsion comprising a polymer and a solvent or a diluent in an amount sufficient for permitting dip forming,
b) providing a form having a first part for forming a contact member adapted to engage with a penis, a second part for forming an opening in the distal end section of the contact member and a third part for forming a discharge conduit, the first, second and third part being integrally connected,
c) dipping said form in said solution or emulsion,
d) removing the form from the solution or emulsion,
e) allowing the solvent or the diluent to evaporate, and
f) optionally, repeating the steps c), d), and e) until the device has attained the desired wall-thickness, and
wherein, prior to dipping according to step c) or during evaporation according to step e), a membrane capable of selectively passing gases, but retaining urine, is arranged at the part of the form for forming the distal part of the contact member or the third part of the form forming the discharge conduit.

During the manufacture it should be ensured that the polymer film resulting from step e) is prevented from coming into contact with the internal and the external face of the membrane or the polymer film is only releaseably attached to the membrane surface to allow the provision of an aperture and a vent on the internal and the external face of the membrane, respectively.

The liquid polymer solution or emulsion for performing the dip forming process according to the invention may comprise any polymer material, which is suitable for engagement with the sensitive skin of the penis. Examples of suitable polymers include SBS, SIS, SEBS, natural rubber latex, SEPS, polyisoprene, polychloroprene, nitril rubber, silicone rubber, curable liquid silicone, polyurethane and any combination and quality thereof.

In the instance the liquid polymer is a solution, the solvent for dissolving the polymer is suitably an inert solvent of an aromatic or aliphatic nature or a combination thereof. However any solvent capable of creating a stable solution may be applied. The amount of solvent is appropriately sufficient for obtaining a solution without precipitations of polymer. In general the amount of solvent is higher than the minimum required for obtaining a solution to obtain a viscosity suitable for dip forming. A preferred amount of solvent is from 400 to 1200 % by weight, based on the amount of polymer.

When the liquid polymer is an emulsion, the polymer is diluted with a suitable diluent. Preferably, the liquid polymer also contains an emulsifying agent to stabilize the emulsion. A suitable diluent is water. It is generally preferred that the emulsion contains sufficient diluent so as to obtain a viscosity of 100 cp to 1000 cp. The emulsion may optionally comprise a suitable coagulation agent, such as Ca(NO₃)₂ or CaCl₂, to obtain an increased wall-thickness. Furthermore, the emulsion may contain a curing agent. The curing agent may e.g. be activated by heat, moisture, ultra violet light, or electron beam. For certain kinds of polymers, e.g. silicone, it is possible to prepare a liquid polymer suitable for dip forming without the usage of a diluent.

The form for dipping into the polymer solution or emulsion is appropriately adapted for receiving a membrane in the part of the form for forming the distal part of the contact member and/or in the third part of the form for forming the discharge conduit. The membrane may according to the invention be provided prior to dipping or during the evaporation.

In case the membrane is provided prior to dipping suitable means is provided for securing that the final device is not covered with a polymer film resulting from the evaporation step e). Various means exist, including providing the intended active membrane area with a coating, which repels the liquid polymer, using a material for the membrane, which in itself repels the polymer solution, or alternatively removing a releasable attached polymer film from the membrane surface with suitable means or covering the intended active membrane area with a removable item.

A preferred method of avoiding a polymer film is to press a central part of the membrane against the form from the external side of the membrane by a suitable tool, which covers the part of the membrane intended to be the active membrane area. While the central part of the membrane area is pressed against the form, edges and corners of the membrane sheet is embedded in the polymer matrix during the dip forming. It may be advantageous to provide the membrane on a suitable carrier, wherein the carrier material surrounds the active membrane area and is adapted to be able to anchor the membrane in the polymer matrix. Thus, the membrane is shielded as the edges and corners is embedded in the polymer matrix, whereby the risk of deteriorating the membrane during use is reduced. Furthermore, a vent passage can be provided by the pressing tool.

It may be preferred to attach the membrane prior to the dipping according to step c) to a raised platform to increase the anchoring effect of the material surrounding the active membrane area and to provide for an aperture having a less tendency to be occluded by the polymer solution during dipping. Preferably, the membrane is kept on the platform during the dip forming procedure by a suitable pressing means, which may allow for the production of a vent passage.

The membrane may also be provided prior to or during evaporation according to step e) or curing if a curing agent is provided in the polymer liquid. Suitably, a sufficient amount of solvent is allowed to evaporate to essentially preserve the dimension stability of an aperture being provided in the polymer film. A membrane sheet comprising an area intended to be active in the final device is arranged, such that it covers the aperture and the area of the membrane surrounding the area intended to be active is attached to the area surrounding the aperture. The attachment can be effected in any appropriate way, e.g. by pressing the surrounding area of the membrane into the still fluid polymer matrix or adhering the surrounding area of the membrane to the area surrounding the aperture by any suitable means following evaporation of an essential amount of the solvent. A shield may be provided aiming at protecting the membrane by a repetition of the steps c), d) and e). It must be secured during the repetition that at least one vent is arranged for allowing gases to be in substantially unhindered contact with the external surface of the membrane. The means for providing the vent can be similar to the means for avoiding the polymer film resulting from the evaporation step e), as discussed above. A preferred method is to press a tool against a central part of the external surface of the membrane to protect the intended active membrane area against coming into contact with the polymer solution.

In another preferred form of the method an external urinary catheter device for the relief of male urinary incontinence is manufactured by a process, comprising the steps of
providing a mould comprising a matrix and a core member defining a mould cavity for plastic injection moulding, wherein the cavity comprising a first part for forming a contact member adapted to engage with a penis, a second part for forming an opening in the distal end section of the contact member and a third part for forming a discharge conduit, said first, second and third parts being integrally connected,
injection of a liquid plastic material into the cavity of the mould,
solidification of the liquid plastic material, and
recovering the moulded device from the mould, and
wherein a membrane capable of selectively passing gases but retaining urine is arranged at the part of the cavity for forming the distal part of the contact member or the third part of the cavity for forming the discharge conduit.

The liquid plastic material for the injection moulding process is typically a melted thermoplastic material, which is solid at normal ambient temperatures or a curable liquid polymeric material such as liquid silicone.

During the injection moulding procedure according to the above process it should be ensured that neither the internal face nor the external face of the membrane is occluded by plastic material. In an aspect of the process of the invention the internal face of the membrane is covering an aperture and/or the external face of the membrane is protected with a shield having a vent.

A vent allowing gases to communicate with the external face of the membrane may be provided by pressing a central part of the membrane against the core by a suitable tool from the matrix side. In addition or alternatively, an aperture allowing urine and gas to communicate with the interior of the membrane surface may be provided by pressing a central part of the membrane against the matrix with a suitable tool from the core side. Suitably, the part of the pressing tool, which faces the membrane, has an area, which may cover a part of the membrane intended to be the active membrane area in the final device. The part of the membrane not pressed with the tool is embedded in the polymer matrix during the plastic injection moulding and provides for an attachment of the membrane to the polymer matrix. To ensure a proper attachment, the membrane may be provided on a carrier having properties, which effectively anchor the membrane to the polymer matrix.

It may be advantageous to design the mould with a core member and/or a matrix, which comprise a raised platform as the tool for pressing the membrane against the matrix and/or the core. In such an embodiment the membrane is arranged within the cavity by pressing the two raised platforms against the membrane. This embodiment ensures the production of a device wherein an aperture as well as a vent is provided. Furthermore, the membrane is shielded from deterioration due to the embedment of the edges and corners of the membrane sheet.

### Brief description of the drawings

Fig. 1 shows a sectional view of an embodiment of the invention with an outer holder member in an application position.
Fig. 2 shows a sectional view of the embodiment depicted in Fig. 1 with the outer holder member in a position of use.
Fig. 3 shows an enlarged view of the embodiment disclosed in Figs. 1 and 2 with the layers in the vicinity of the membrane partly broken away.
Fig. 4 shows a detail of a second embodiment of the invention with the shield partly broken away.
Fig. 5 shows a sectional view of the detail of the second embodiment in Fig. 4. The section is indicated by V in Fig. 4.
Fig. 6 shows a front view of a detail of a third embodiment of the invention.
Fig. 7 shows a sectional view of the detail of the embodiment of Fig. 6. The section is indicated by VII in Fig. 6.
Fig. 8 shows a sectional view of a detail of the embodiment shown on Figs. 6 and 7. The section is indicated by VIII in Fig. 6.
Fig. 9 shows a front view of a detail of a fourth embodiment of the invention.
Fig. 10 shows a sectional view of a detail of the embodiment indicated in Fig. 9. The section is shown by X in Fig. 9.
Fig. 11 shows a sectional view of a detail of the embodiment shown in Figs. 9 and 10. The section is prepared as indicated by XI in Fig. 9.
Fig. 12 shows a detail of a front view of a fifth embodiment of the invention.
Fig. 13 shows a sectional view of a detail of the embodiment shown in Fig. 12. The section is indicated in Fig. 12 at XIII.
Fig. 14 shows a detail of a front view of an embodiment with a ledge that provides for fastening of the tubular member in an axial direction.
Fig. 15 shows a sectional view of a detail of the embodiment shown in Fig. 14.
Fig. 16 shows a detail of a front view of an embodiment, wherein the fastening means is provided by a point welding.
Fig. 17 shows a detail in perspective, wherein vents are provided in the interior face of the tubular member.
Fig. 18 shows a detail of a front view, wherein a membrane device fastens the tubular member to the discharge conduit.
Fig. 19 shows a sectional view of the embodiment shown in Fig. 18. The section is indicated in Fig. 18 at XIX.

### Detailed description of the invention

In the various figures of the drawing identical parts of various embodiments of the external urinary catheter device of the invention of parts having the same function have been indicated by identical reference numerals.

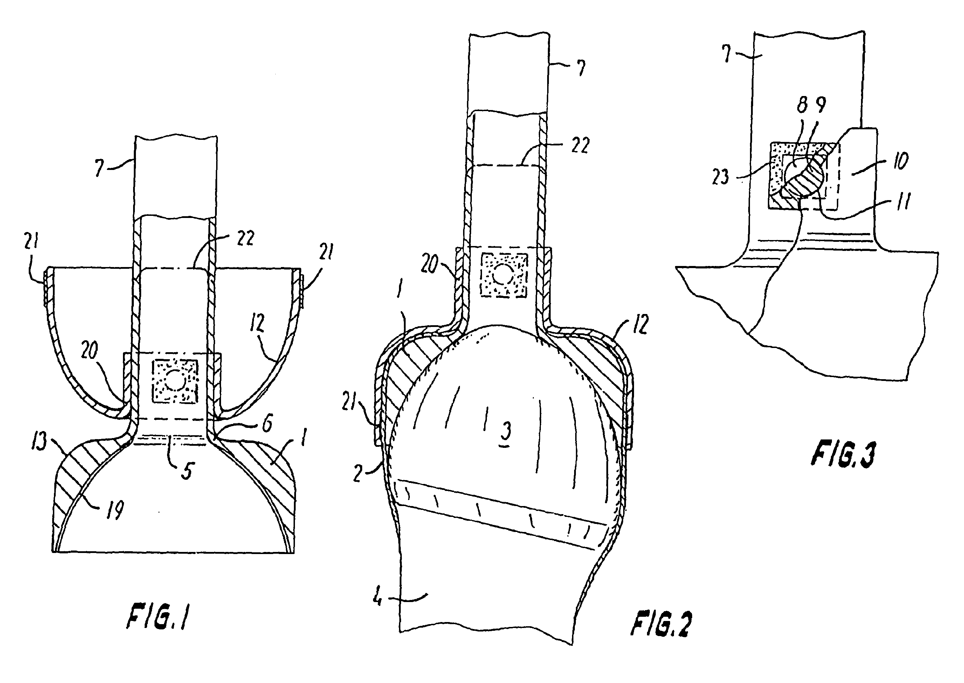

The catheter device shown in Figs. 1, 2, and 3 comprises a contact member 1 formed with an opening 5, which in a position of use as shown in Fig. 2 is situated opposite the urethral orifice in the corona 3 of a penis 4. A distal end section 6 of the contact member 1 surrounds the opening 5 and joins a discharge conduit 7 by which the catheter may be connected e.g. with a hose member, through which discharges of urine may flow from the contact member into a collection bag not illustrated. Thereby, a sealed urine flow passage is provided to extend in a substantially axial direction from the inner side of the contact member 1 facing the urethral orifice towards the exterior of the catheter device.

As shown in Fig. 2 the contact member 1 is formed to be placed in use under the foreskin 2 of the penis 4 in contact with the corona 3.

In the distal end section 6 around the discharge conduit 7 the contact member 1 is designed with increased form stability so as to form a shoulder-like ledge 13 having e.g. a substantially flat end face, so that in use the contact member 1 is retained by the foreskin 2.

To provide an improved sealing effect against the corona 3 the inner side of the contact member 1 is coated with a layer 19 of a skin-friendly adhesive as used for medical devices intended to be in contact with sensitive skin tissue.

In the embodiment shown, the discharge conduit 7 and the contact member 1 are connected with a surrounding outer holder member 12 having an opening for the discharge conduit 7 via the tubular connection member 20. As shown in Figs 1 and 2, the outer holder member 12 is formed as an invertible substantially bowl-shaped member, which from the inverted application position in Fig. 1 can be brought into the position of use by tilting it around the transition to the connection member 20. An annular applicator not shown in the figures for engagement with the extreme portions of the outer holder member 12 may be used to invert the outer holder member 12 from the application position depicted in Fig. 1 to the position of use depicted in Fig. 2. Such an annular applicator will be effective to facilitate accurate positioning of the contact member 1 relative to the corona during application of the catheter device.

The inner engagement surface of the outer holder member 12 is partly or totally coated with a layer 21 of a skin-friendly adhesive for improved engagement with the external surface of the foreskin 2. The provision of the adhesive is not compulsory, however, and may be dispensed with, since a sufficient holding force may be obtained by other means, e.g. dimensioning of the outer holder member with a length so as to cover, in the position of use, the entire external side of the foreskin surrounding the corona.

In order to facilitate removal of the catheter device after use the distal end of the tubular connection member 20 is formed with a projecting flap 22, which may serve as a grip which can be operated to remove the outer holder member 12 by pulling back the projecting flap 22 in a direction towards the inverted position, from which the holder member was originally applicated.

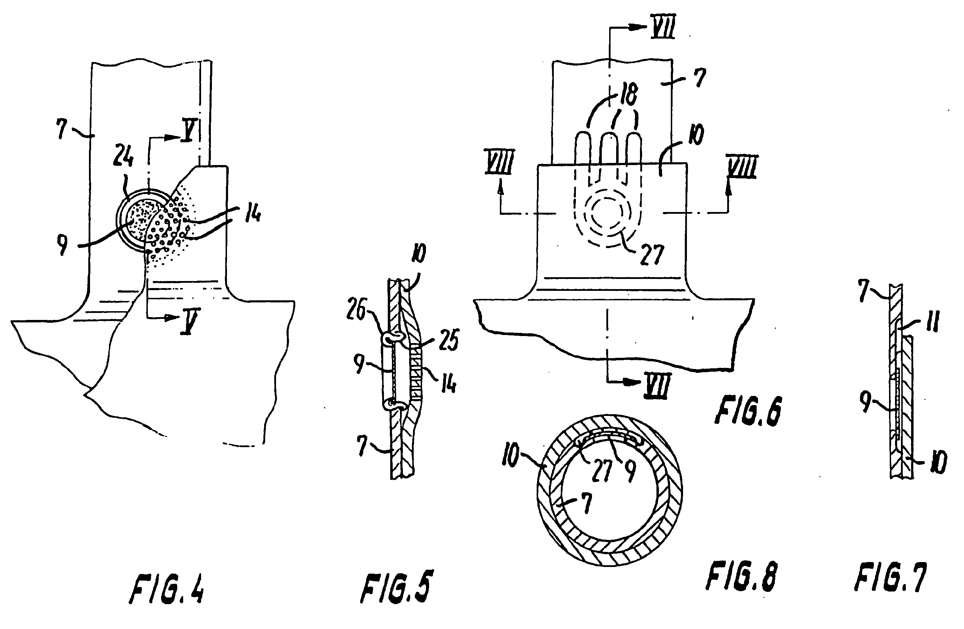

As shown in detail in Fig. 3 an aperture 8 is provided in a proximal part of the discharge conduit 7 relatively close to the urethral orifice in a position of use. A membrane 9 capable of selectively passing gases, but retaining urine, is covering the aperture 8 and attached to the surface area of the discharge conduit surrounding the aperture 8 by means of an adhesive 23. The membrane 9 is in turn covered with a shield 10, which is a part of the tubular connection member 20, for providing protection of the membrane. A single vent 11 is provided in the shield 10 for allowing gases to be in substantially unhindered contact with the external surface of the membrane. The vent 11 in the shield 10 has a dimension corresponding to the dimension of the aperture 8 allowing the active membrane area virtually unhindered to communicate with the surrounding environment, while preventing the edges and corners of the membrane to be exposed to mechanical wear, thus reducing the risk of deterioration of the membrane.

Figs. 4 and 5 show an embodiment of the invention using a rivet 24 to fasten the membrane to an area of the discharge conduit surrounding the aperture. The membrane is fastened to the orifice of the rivet by clamping between the male 25 and the female 26 part of the rivet 24. The male part 25 of the rivet 24 shown on Fig. 5 provides a raised platform for the shield 10, whereby an increased clearance between the external side of the membrane and the shield is formed. The shield is provided with a plurality of apertures 14 as vents allowing a virtually unimpeded diffusion of gases between the space above the membrane surface and the surrounding environment.

Figs. 6, 7, and 8 show an embodiment of the invention where the membrane is depressed in the surface of the discharge conduit 7 and tracks 18 are provided for ventilation. A hand-like depression is cast in the outer face of the discharge conduit, said depression comprising a carpus 27 and three finger-like tracks 18 extending in a distal direction. The carpus 27 of the hand-like depression is provided with a membrane 9 attached to the area around the aperture by welding. A tubular shield 10 is covering the carpus 27 and a part of the finger-like tracks 18 but not the fingertips. During use of the catheter device of the invention air may be vented through the openings at the fingertips and conducted via the fingers covered by the shield 10 to the external surface of the membrane 9.

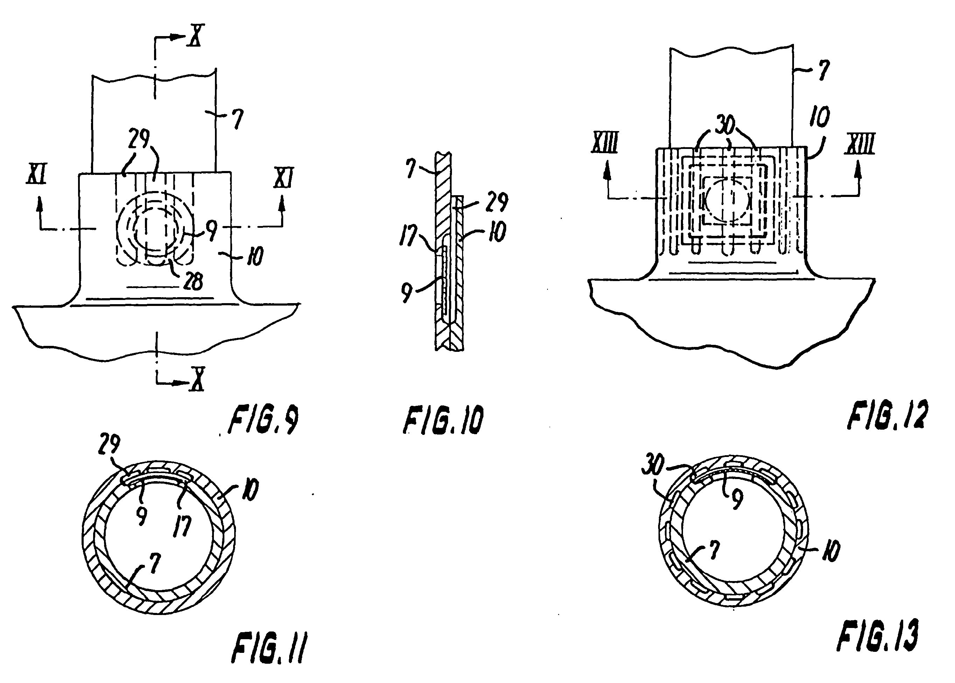

Figs. 9, 10, and 11 disclose an embodiment of the invention, where the membrane is depressed in the surface of the discharge conduit and tracks in the inner face of the shield provide for the ventilation. A circular depression 28 is formed in the discharge conduit 7 in an area around the circular aperture 8. A membrane 9 is attached to the depressed area around the aperture by welding. A tubular shield 10 is covering the depression and extents over the circumference thereof. Three tracks 29 are provided in the inner face of the shield 10 to establish a vent passage from the external surface of the membrane to the exterior of the catheter device. In operation, air is sucked in at the opening formed by the tracks 29 in the edge of the shield 10. The air is subsequently conducted through the tracks to the external surface of the membrane.

Figs. 12 and 13 show an embodiment of the invention, in which the vent passage is provided by a plurality of tracks formed in the inner face of the shield. The membrane 9 is provided and covers the aperture as explained for the embodiment according to Figs. 1, 2, and 3. A tubular shield 10 is surrounding the part of the discharge conduit harbouring the aperture covered with the membrane. The edge of the tubular shield extends above the upper edge of the membrane. A plurality of tracks 30 substantially parallel to the axis of the discharge conduit is formed in the inner face of the shield in the entire circumference thereof. In operation, air is sucked in at the openings formed by the tracks 30 in the edge of the shield 10 and subsequently conveyed to the external surface of the membrane. Thus, the tracks 30 establish a vent passage allowing air from the exterior of the catheter to come into contact with the surface of the membrane.

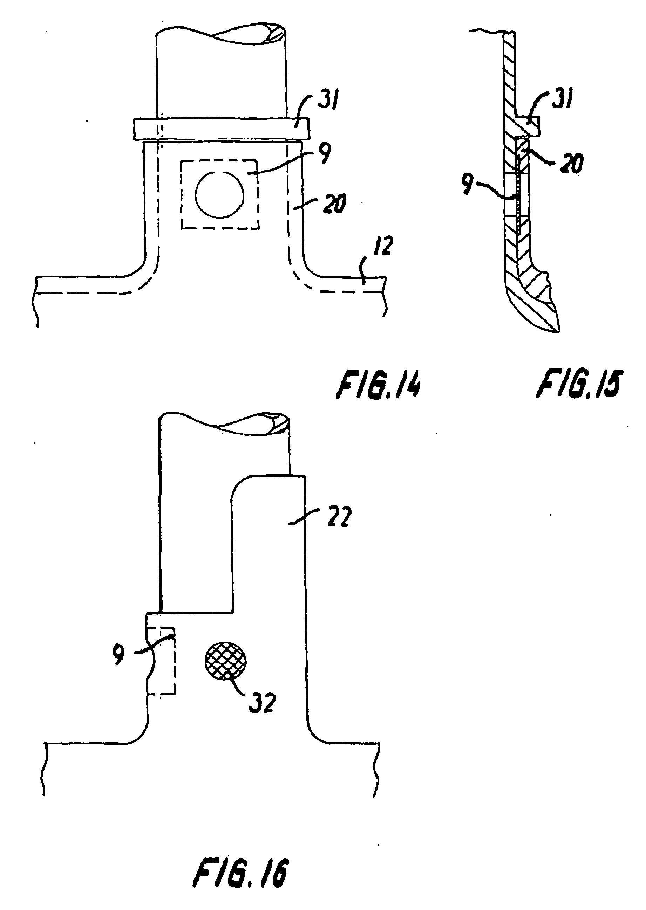

Figs. 14 and 15 show an embodiment of the invention, in which the outer holder member 12 is impeded from moving in an axial direction when stressed with a forward or backward pulling force. When the outer holder member is pulled forward, that is towards the penis e.g. during application, the outer holder member is halted against axial movement by the contact member and during use the tubular connection member connected with the outer holder member is halted against axial movement by the ledge 31 provided on the discharge conduit 7 just above the upper edge of the tubular member. This embodiment is of special interest, when the adherence between the inner face of the tubular connection member and the external face of the proximal part of the discharge conduit is absent or reduced, e.g. due to lack of or reduced inherent adherence between the membrane and the inner face of the tubular connection member.

In the embodiment shown in Fig. 16 the outer holder member and the inner member is fixed in an axial as wells a rotational direction by an ultrasonic point welding 32. The point welding is conducted in an area, wherein the membrane 9 is not sandwiched. Another point welding not shown in the figure is provided on the diagonally opposed face of the device. The embodiment is provided with a single projecting flap 22. Alternative means of fixing the outer holder member with respect of the inner member could be foreseen such as use of a membrane device arranged in an aperture going through the tubular member as well as the proximal part of the discharge conduit

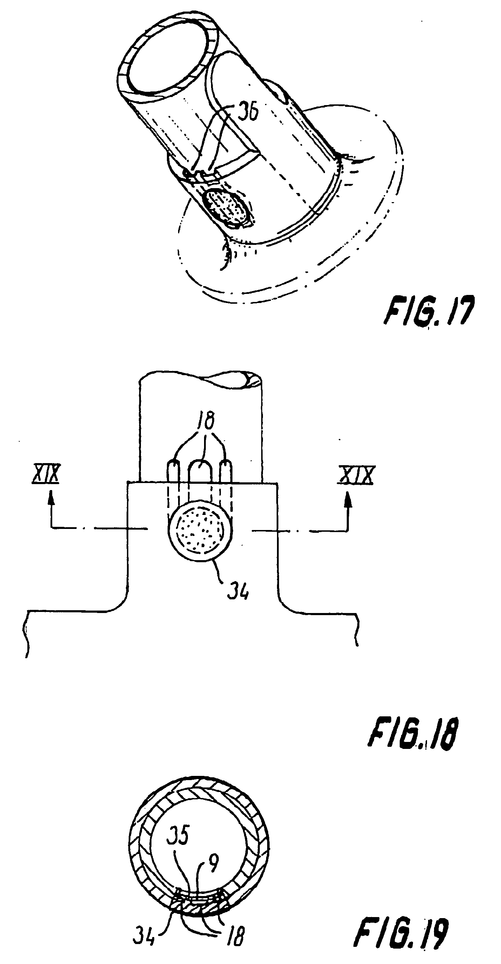

Fig. 17 discloses a detail of an embodiment where a tubular member covering the entire surface of the membrane shields the membrane. For allowing gases to communicate with the exterior of the membrane, tracks are provided in the inner face of the tubular member.

Figs. 18 and 19 depict an embodiment where the tubular connection member is fastened to the discharge conduit by use of a membrane device 34. The membrane device comprises a housing and a membrane 9, the membrane being supported by corrugations 35. The corrugations provide for tracks, which are extended in a part of the discharge conduit. The membrane is placed in an aperture going through the tubular connection member as well as the proximal part of the discharge conduit securing a locked position in an axial and a rotational direction of the outer holder member relative to the contact member. Finger-like cannels 18 are formed in the external face of the discharge conduit to provide for gas communication between the external surface of the membrane and the surrounding air. The finger-like channels are partly covered by the tubular connection member, whereby the fingertips are not covered, however, and secure a proper ventilation.

While in the foregoing, embodiments of the invention have been disclosed in considerable detail for purpose of illustration, it will be understood by those skilled in the art that many of these details may be varied without departing from the scope of the invention. Thus, individual features of the various described embodiments may to a wide extent be interchanged and the provision of an outer holder member as disclosed in the described embodiment may be dispensed with as known per se, e.g. from EP-A-0 706 355.

## Claims

1. An external urinary catheter device for the relief of male urinary incontinence, comprising
a contact member (1) adapted for arrangement, in a position of use, in engagement with at least an extreme portion (3) of a penis (4)
an opening (5) formed in a distal end section (6) of the contact member (1) to be positioned substantially opposite the urethral orifice in the position of use,
a discharge conduit (7) connected with the opening (5) to provide a sealed urine flow passage extending in a substantial axial direction towards the exterior of the catheter device,
an aperture (8) provided in a distal part of the contact member or in the discharge conduit (7),
a membrane (9) attached to the surface surrounding said aperture (8) and capable of selectively passing gases but retaining urine, **characterised by**
an external protective shield (10) arranged for at least partly covering the membrane, and
at least one vent (11, 14, 18, 29, 30) provided for allowing gases to be in substantially unhindered contact with the external surface of the membrane.

2. The external urinary catheter device according to claim 1, wherein the contact member (1) is integrally connected with discharge conduit (7) via the opening (5) formed in the distal end section of the contact member (1).

3. The external urinary catheter device according to claim 1 or 2, wherein the contact member (1) is arrangeable, in a position of use, between the foreskin (2) and the corona (3) of a penis (4).

4. The external urinary catheter device according to claim 3, wherein the contact member (1) comprises a shoulder-like ledge (13) at a substantially plane abutment face of the contact member (1) around and substantially perpendicular to the discharge conduit (7).

5. The external urinary catheter device according to claim 3, wherein the contact member (1) comprises a plurality of leaves comprised of a film having an adhesive layer on the interior surface thereof to secure an attachment of the device to the corona of a penis.

6. The external urinary catheter device according to any of the preceding claims, wherein the contact member (1) constitutes an inner member of the catheter device, the catheter device further comprising an outer holder member (12) for retaining the contact member (1) in the position of use, said outer holder member (12) being formed in a distal end section thereof with an opening for said discharge conduit (7) and being arrangeable, in said position of use, in a substantially fixed axial position with respect to the discharge conduit (7) for circumferential engagement with the external side of the foreskin (3).

7. The external urinary catheter device according to any of the preceding claims, wherein the membrane (9) is attached to said surrounding surface by means of an adhesive (23).

8. The external urinary catheter device accord- 30 ing to any of claims 1 to 6, wherein the membrane (9) is attached to said surrounding surface by means of a rivet member (24).

9. The external urinary catheter device according to any of claims 1 to 6, wherein the membrane (9) is attached to said surrounding surface by welding.

10. The external urinary catheter device according to any of claims 1 to 9, wherein the membrane (9) is adapted to allow passage of urine at pressures more than 80 mbar above ambient pressure.

11. The external urinary catheter device according to any of the preceding claims, wherein said aperture (8) is provided in the discharge conduit (7) of the contact member (1) and said shield (10) comprises a tubular member (20) surrounding the discharge conduit (7) to cover said aperture (8) and the membrane (9) attached to said surrounding surface.

12. The external urinary catheter device according to claims 6 and 11, wherein said tubular member (20) forms part of the outer holder member (12).

13. The external urinary catheter device according to claim 12, wherein the outer holder member (12) is fixed in at least an axial direction with respect to the inner member (1) by fastening means.

14. The external urinary catheter device according to claim 13, wherein the outer holder member (12) is fixed in an axial direction as well as a rotational direction with respect to the inner member (1) by fastening means.

15. The external urinary catheter device according to claim 13 or 14, wherein said tubular member (20) forms an integral part of said outer holder member (12) and is connected by said fastening means with a proximal part of the discharge conduit (7) of the inner member (1).

16. The external urinary catheter device according to claim 15, wherein said fastening means comprises one or more ledges (31) in the inner face of the tubular member (20) and/or the outer face of the discharge conduit (7).

17. The external urinary catheter device according to claim 15, wherein said fastening means comprises a membrane device (34) arranged in an aperture going through said tubular member (20) as well as said proximal part of the discharge conduit (7).

18. The external urinary catheter device according to any of claims 13 to 15, wherein said fastening means comprises a thermal or an ultrasonic welding bond.

19. The external urinary catheter device according to any of the preceding claims, wherein said vent comprises at least one opening (11, 14) in said shield (10) in flow communication with the external surface of the membrane (9).

20. The external urinary catheter device according to any of the preceding claims, wherein the membrane (9) is depressed with respect to said surrounding surface to provide a clearance with respect to said shield (10) and said vent comprises at least one track (18, 29, 30) formed by a depression in an internal surface of said shield (10) and/or said surrounding surface part to provide a gas flow communication between said clearance and the exterior of the catheter device.

21. A method for production of an external urinary catheter device for the relief of male urinary incontinence, which comprises the steps of: providing a catheter part comprising a contact member (1) which is adapted for engagement with at least an extreme portion (3) of a penis (4), said contact member comprising a distal end section (6) providing an opening (5) located to be positioned substantially opposite the urethral orifice by said engagement of the contact member (1) with said extreme portion (3) and a discharge conduit (7) connected with the opening (5), providing an aperture (8) in a distal part of the contact member (1) or in the discharge conduit (7) attaching a membrane (9) to the surface surrounding said aperture (a), the membrane being capable of selectively passing gases but retaining urine, **characterised by** providing a protective shield (10) for at least partly covering the membrane, and arranging at least one vent (11, 14, 18, 29, 30) for allowing gases to be in substantially unhindered contact with the external surface of the membrane.

22. The method according to claim 21, wherein said aperture (8) is provided in the discharge conduit (7) of the contact member (1) and said shield (10) comprises a tubular member (20) and is provided to surround the discharge conduit (7) to cover said aperture (8) and the membrane (9) attached to said surrounding surface.

23. The method according to claim 21 or 22, further comprising the step of providing the catheter part with an outer holder member (12) for retaining the contact member (1) in a position of use as an inner member between the foreskin (2) and the corona (3) of a penis (4), said outer holder member (12) being formed in a distal end section with an opening for said discharge conduit (7) and being adapted for arrangement, in a position of use, in a substantially fixed axial position with respect to the discharge conduit (7) for circumferential engagement with the external side of the foreskin (2).

24. The method according to claims 22 and 23, wherein said tubular member (20) is formed as part of the outer holder member (12).

25. The method according to claim 24, wherein the outer holder member (12) is provided to be fixed in at least an axial direction with respect to the inner member (1) by fastening means.

26. The method according to claim 25, wherein said tubular member (20) is formed as an integral part of said outer holder member (12) and said fastening means is provided to connect said tubular member (20) with a proximal part of the discharge conduit (7) of the inner member (1).

27. The method according to claim 25 or 26, wherein said fastening means is provided by a thermal or an ultrasonic welding.

28. The method according to any of claims 21 to 27, wherein said catheter part is provided by a process comprising the steps of a) providing a liquid polymer solution or emulsion comprising a polymer and a solvent or a diluent in an amount sufficient for permitting dip forming, b) providing a form having a first part for forming said contact member, a second part for forming said opening in the distal end section of the contact member and a third part for forming said discharge conduit, said first, second and third parts being integrally connected, c) dipping said form in said solution or emulsion, d) removing the form from the solution or emulsion, e) allowing the solvent or the diluent to evaporate, and f) optionally, repeating the steps c), d), and e) until the device has attained a desired wallthickness, and wherein, prior to dipping according to step c) or during evaporation according to step e), said membrane is arranged at the part of the form for forming the distal part of the contact member or at the third part of the form for forming the discharge conduit.

29. The method according to any of claims 21 to 27, wherein said catheter part is provided by a process comprising the steps of providing a mould comprising a matrix and a core member defining a mould cavity for plastic injection moulding, said cavity comprising a first part for forming said contact member, a second part for forming said opening in the distal end section of the contact member and a third part for forming said discharge conduit, said first, second and third parts being integrally connected, injection of a liquid plastic material into said mould cavity, solidification of the liquid plastic material, and recovering the moulded device from the mould, and wherein said membrane is arranged at the part of the cavity for forming the distal part of the contact member or the third part of the cavity for forming the discharge conduit.

## Patentansprüche

1. Externe Harnkathetervorrichtung zur Abhilfe bei männlicher Harninkontinenz, die aufweist:
ein Kontaktelement (1), das, in einer Anwendungsposition, zur Anordnung in Anlage an zumindest einem äußersten Teil (3) eines Penis (4) ausgebildet ist,
eine Öffnung (5), die in einem distalen Endabschnitt (6) des Kontaktelements (1) ausgebildet ist und in der Anwendungsposition im Wesentlichen gegenüber der Harnröhrenöffnung anzuordnen ist,
eine Abführungsleitung (7), die mit der Öffnung (5) verbunden ist und eine dichte Urinabflussleitung bildet, die sich in einer im Wesentlichen axialen Richtung zur Außenseite der Kathetervorrichtung hin erstreckt,
eine Öffnung (8), die in einem distalen Teil des Kontaktelements oder in der Abführungsleitung (7) vorgesehen ist,
eine Membran (9), die an der die Öffnung (8) umgebenden Fläche angebracht und befähigt ist, Gase selektiv durchzulassen, aber Urin zurückzuhalten,
**gekennzeichnet durch**
eine äußere Schutzhülle (10), die so ausgebildet ist, dass sie die Membran zumindest teilweise abdeckt, und
mindestens einen Gasauslass (11, 14, 18, 29, 30), der dazu vorgesehen ist, dass Gase in im Wesentlichen ungehindertem Kontakt mit der Außenoberfläche der Membran stehen können.

2. Externe Harnkathetervorrichtung nach Anspruch 1, bei der das Kontaktelement (1) über die Öffnung (5), die im distalen Endabschnitt des Kontaktelements (1) ausgebildet ist, integral mit der Abführungsleitung (7) verbunden ist.

3. Externe Harnkathetervorrichtung nach Anspruch 1 oder 2, bei der das Kontaktelement (1), in einer Anwendungsposition, zwischen der Vorhaut (2) und der Corona (3) eines Penis (4) anzuordnen ist.

4. Externe Harnkathetervorrichtung nach Anspruch 3, bei der das Kontaktelement (1) an einer im Wesentlichen ebenen Angrenzfläche des Kontaktelements (1) um die Abführungsleitung (7) herum und senkrecht zu ihr einen schulterartigen Vorsprung (13) aufweist.

5. Externe Harnkathetervorrichtung nach Anspruch 3, bei der das Kontaktelement (1) mehrere Flügel aus einem Film mit einer Klebeschicht auf ihrer Innenoberfläche zur sicheren Anbringung der Vorrichtung an der Corona eines Penis aufweist.

6. Externe Harnkathetervorrichtung nach einem der vorhergehenden Ansprüche, bei der das Kontaktelement (1) ein inneres Element der Kathetervorrichtung darstellt, wobei die Kathetervorrichtung ferner ein äußeres Halteelement (12) zum Halten des Kontaktelements (1) in der Anwendungsposition aufweist, wobei das äußere Halteelement (12) in einem distalen Endabschnitt davon mit einer Öffnung für die Abführungsleitung (7) ausgebildet ist und, in der Anwendungsposition, in einer im Wesentlichen festen axialen Position in Bezug auf die Abführungsleitung (7) zur Anlage in Umfangsrichtung an der Außenseite der Vorhaut (3) anzuordnen ist.

7. Externe Harnkathetervorrichtung nach einem der vorhergehenden Ansprüche, bei der die Membran (9) mittels eines Klebers (23) an der umgebenden Fläche angebracht ist.

8. Externe Harnkathetervorrichtung nach einem der Ansprüche 1 bis 6, bei der die Membran (9) mittels eines Nietelernents (24) an der umgebenden Fläche angebracht ist.

9. Externe Harnkathetervorrichtung nach einem der Ansprüche 1 bis 6, bei der die Membran (9) durch Schweißen an der umgebenden Fläche angebracht ist.

10. Externe Harnkathetervorrichtung nach einem der Ansprüche 1 bis 9, bei der die Membran (9) so ausgebildet ist, dass sie einen Durchtritt von Urin bei Drucken von mehr als 80 mbar über Umgebungsdruck erlaubt.

11. Externe Harnkatkretervorrichtung nach einem der vorhergehenden Ansprüche, bei der die Öffnung (8) in der Abführungsleitung (7) des Kontaktelements (1) vorgesehen ist und die Schutzhülle (10) ein rohrförmiges Element (20) aufweist, das die Abführungsleitung (7) umgibt und die Öffnung (8) sowie die an der umgebenden Fläche angebrachte Membran (9) abdeckt.

12. Externe Harnkathetervorrichtung nach Anspruch 6 und 11, bei der das rohrförmige Element (20) einen Teil des äußeren Halteelements (12) bildet.

13. Externe Harnkathetervorrichtung nach Anspruch 12, bei der das äußere Halteelement (12) durch Befestigungsmittel in zumindest einer axialen Richtung in Bezug auf das innere Element (1) befestigt ist.

14. Externe Harnkathetervorrichtung nach Anspruch 13, bei der das äußere Halteelement (12) durch Befestigungsmittel in einer axialen Richtung sowie in einer Drehrichtung in Bezug auf das innere Element (1) befestigt ist.

15. Externe Harnkathetervorrichtung nach Anspruch 13 oder 14, bei der das rohrförmige Element (20) einen integralen Teil des äußeren Halteelements (12) bildet und durch die Befestigungsmittel mit einem proximalen Teil der Abführungsleitung (7) des inneren Elements (1) verbunden ist.

16. Externe Harnkathetervorrichtung nach Anspruch 15, bei der die Befestigungsmittel einen oder mehrere Vorsprünge (31) in der Innenfläche des rohrförmigen Elements (20) und/oder der Außenfläche der Abführungsleitung (7) umfassen.

17. Externe Harnkathetervorrichtung nach Anspruch 15, bei der die Befestigungsmittel eine Mernbranvorrichtung (34) umfassen, die in einer Öffnung angeordnet ist, die durch das rohrförmige Element (20) sowie den proximalen Teil der Abführungsleitung (7) hindurchgeht.

18. Externe Harnkathetervornchtung nach einem der Ansprüche 13 bis 15, bei der die Befestigungsmittel eine thermisch oder durch Ultraschall erzeugte Schweißverbindung umfassen.

19. Externe Harnkathetervorrichtung nach einem der vorhergehenden Ansprüche, bei welcher der Gasauslass mindestens eine Öffnung (11, 14) in der Schutzhülle (10) in Fließverbindung mit der Außenfläche der Membran (9) umfasst.

20. Externe Harnkathetervorrichtung nach einem der vorhergehenden Ansprüche, bei der die Membran (9) in Bezug auf die umgebende Fläche vertieft liegt und so einen freien Raum in Bezug auf die Schutzhülle (10) bildet und der Gasauslass mindestens einen Kanal (18, 29, 30) umfasst, der durch eine Vertiefung in einer Innenfläche der Schutzhülle (10) und/oder den Teil der umgebenden Fläche gebildet wird und eine Gas-Fließverbindung zwischen dem freien Raum und dem Äußeren der Kathetervorrichtung ergibt.

21. Verfahren zur Herstellung einer externen Harnkathetervorrichtuxzg zur Abhilfe bei männlicher Harninkontinenz, das folgende Schritte umfasst:
Vorsehen eines Katheterteils, das ein Kontaktelement (1) aufweist, das zur Anlage an zumindest einem äußersten Teil (3) eines Penis (4) ausgebildet ist, wobei das Kontaktelement einen distalen Endabschnitt (6) mit einer Öffnung (5), die so angeordnet ist, dass sie durch die Anlage des Kontaktelements (1) an dem äußersten Teil (3) im Wesentlichen gegenüber der Harnrökarenöffnung anzuordnen ist, und eine Abführungsleitung (7) aufweist, die mit der Öffnung (5) verbunden ist,
Vorsehen einer Öffnung (8) in einem distalen Teil des Kontaktelements (1) oder in der Abfülxxungsleitung (7),
Anbringen einer Membran (9) an der die Öffnung (8) umgebenden Fläche,
wobei die Membran befähigt ist, Gase selektiv durchzulassen, aber Urin zurückzuhalten,
**gekennzeichnet durch**
Vorsehen einer Schutzhülle (10) zur mindestens teilweisen Abdeckung der Membran
und
Ausbilden mindestens eines Gasauslasses (11, 14, 18, 29, 30), der es erlaubt, dass Gase in im Wesentlichen ungehindertem Kontakt mit der Außenfläche der Membran stehen können.

22. Verfahren nach Anspruch 21, bei dem die Öffnung (8) in der Abführungsleitung (7) des Kontaktelements (1) vorgesehen wird und die Schutzhülle (10) ein rohrförmiges Element (20) aufweist und so ausgebildet ist, dass sie die Abflussleitung (7) umgibt und die Öffnung (8) sowie die Membran (9), die an der umgebenden Fläche angebracht ist, abdeckt.

23. Verfahren nach Anspruch 21 oder 22, das ferner folgenden Schritt umfasst:
Versehen des Katheterteils mit einem äußeren Halteelement (12) zum Halten des Kontaktelements (1) in einer Anwendungsposition als inneres Element zwischen der Vorhaut (2) und der Corona (3) eines Penis (4), wobei das äußere Halteelement (12) in einem distalen Endabschnitt davon mit einer Öffnung für die Abführungsleitung (7) gebildet ist und so ausgebildet ist, dass es, in einer Awerzdungsposition, in einer im Wesentlichen festen axialen Position in Bezug auf die Abführungsleitung (7) zur Anlage in Umfangsrichtung an der Außenseite der Vorhaut (3) anzuordnen ist.

24. Verfahren nach Anspruch 22 und 23, wobei das rohrförmige Element (20) als Teil des äußeren Halteelements (12) ausgebildet wird.

25. Verfahren nach Anspruch 24, bei dem das äußere Halteelement (12) so vorgesehen wird, dass es in mindestens einer axialen Richtung in Bezug auf das innere Element (1) durch Befestigungsmittel befestigt wird.

26. Verfahren nach Anspruch 25, bei dem das rohrförmige Element (20) als integraler Teil des äußeren Halteelements (12) ausgebildet wird und die Befestigungsmittel so vorgesehen werden, dass das rohrförmige Element (20) mit einem proximalen Teil der Abführungsleitung (7) des inneren Elements (1) verbunden wird.

27. Verfahren nach Anspruch 25 oder 26, bei dem die Befestigungsmittel durch thermisches Schweißen oder Ultraschallschweißen gebildet werden.

28. Verfahren nach einem der Ansprüche 21 bis 27, wobei das Katheterteil durch ein Verfahren erzeugt wird, das folgende Schritte umfasst:
a) Vorsehen einer flüssigen Polymerlösung oder Polymeremulsion, die ein Polymer und ein Lösungsmittel oder ein Verdünnungsmittel in einer Menge enthält, die ausreicht, um ein Tauchformen zu ermöglichen,
b) Vorsehen einer Form mit einem ersten Teil zur Ausformung des Kontaktelements, einem zweiten Teil zur Ausformung der Öffnung im distalen Endabschnitt des Kontaktelements und einem dritten Teil zur Ausformung der Abführungsleitung,
wobei der erste Teil, der zweite Teil und der dritte Teil integral miteinander verbunden sind,
c) Eintauchen der Form in die Lösung oder die Emulsion,
d) Entfernen der Form aus der Lösung oder der Emulsion,
e) Verdampfenlassen des Lösungsmittels oder Verdünnungsmittels,
und
f) wahlweise Wiederholen der Schritte c), d) und e), bis die Vorrichtung eine gewünschte Wandstärke erreicht hat, und wobei, vor dem Eintauchen gemäß Schritt c) oder während des Verdampfens gemäß Schritt e), die Membran an dem zur Ausformung des distalen Teils des Kontaktelements dienenden Teil der Form oder an dem zur Ausformung der Abführungsleitung dienenden dritten Teil der Form angeordnet wird.

29. Verfahren nach einem der Ansprüche 21 bis 27, wobei das Katheterteil durch ein Verfahren erzeugt wird, das folgende Schritte umfasst:
Vorsehen einer Form, die eine Matrix und ein Kernelement aufweist, die einen Formhohlraum zum Kunststoffspritzgießen vorgeben,
wobei der Formhohlraum einen ersten Teil zur Ausformung des Kontaktelements, einen zweiten Teil zur Ausformung der Öffnung im distalen Endabschnitt des Kontaktelements und einen dritten Teil zur Ausformung der Abführungsleitung aufweist, und wobei der erste Teil, der zweite Teil und der dritte Teil integral miteinander verbunden sind,
Einspritzen eines flüssigen Kunststoffmaterials in den Formhohlraum,
Verfestigenlassen des flüssigen Kunststoffmaterials
und
Entnahme der ausgeformten Vorrichtung aus der Form, und wobei die Membran an dem zur Ausformung des distalen Teils des Kontaktelements dienenden Teil des Formhohlraums oder an dem zur Ausformung der Abführungsleitung dienenden dritten Teil des Formhohlraums angeordnet wird.

## Revendications

1. Dispositif de cathéter urinaire à implantation externe pour remédier à l'incontinence urinaire masculine, comprenant
un élément de contact (1) agencé pour être placé, en position d'utilisation, en prise d'engagement avec au moins la partie extrême (3) d'un pénis (4),
une ouverture (5) ménagée dans la portion d'extrémité distale (6) de l'élément de contact (1) destinée à être positionnée sensiblement en face de l'orifice de l'urètre en position d'utilisation,
un conduit d'évacuation (7) raccordé à l'ouverture (5) destiné à constituer un passage étanche pour l'écoulement de l'urine, s'étendant dans une direction sensiblement axiale vers l'extérieur du dispositif de cathéter,
un orifice (8) ménagé dans la partie distale de l'élément de contact ou dans le conduit d'évacuation (7),
une membrane (9) fixée sur la surface entourant ledit orifice (8) et capable de manière sélective de laisser passer les gaz mais de retenir l'urine,
**caractérisé en ce qu'**il comporte un écran de protection extérieur (10) disposé de manière à recouvrir au moins de manière partielle la membrane et au moins un dispositif d'ouverture (11, 14, 18, 29, 30) prévu pour permettre aux gaz d'être pratiquement librement en contact avec la surface extérieure de la membrane.

2. Dispositif de cathéter urinaire à implantation externe selon la revendication 1, dans lequel l'élément de contact (1) est intégralement raccordé au conduit d'évacuation (7) par l'intermédiaire de l'ouverture (5) ménagée dans la portion d'extrémité distale (6) de l'élément de contact (1).

3. Dispositif de cathéter urinaire à implantation externe selon la revendication 1 ou 2, dans lequel l'élément de contact (1) est agencé pour pouvoir être disposé, en position d'utilisation, entre le prépuce (2) et la gaine fibreuse (3) du pénis (4).

4. Dispositif de cathéter urinaire à implantation externe selon la revendication 3, dans lequel l'élément de contact (1) comprend une saillie en forme d'épaulement (13) au niveau de la face de contact de butée sensiblement plane de l'élément de contact (1) autour du et sensiblement perpendiculaire au conduit d'évacuation (7).

5. Dispositif de cathéter urinaire à implantation externe selon la revendication 3, dans lequel l'élément de contact (1) comprend une pluralité de feuilles constituées par une pellicule comprenant une couche adhésive sur sa surface intérieure pour assujettir le moyen de fixation du dispositif sur la gaine fibreuse du pénis.

6. Dispositif de cathéter urinaire à implantation externe selon l'une quelconque des revendications précédentes, dans lequel l'élément de contact (1) constitue l'élément intérieur du dispositif de cathéter, le dispositif de cathéter comprenant en outre un élément formant support extérieur (12) pour maintenir l'élément de contact (1) dans sa position d'utilisation, ledit élément formant support extérieur (12) se présentant sous la forme d'un segment d'extrémité distant de celui-ci, muni d'une ouverture pour le passage dudit conduit d'évacuation (7) et pouvant être disposé, dans ladite position d'utilisation, dans une position axiale sensiblement fixe par rapport au conduit d'évacuation (7) pour être mise en prise d'engagement au niveau de sa circonférence avec la surface extérieure du prépuce (3).

7. Dispositif de cathéter urinaire à implantation externe selon l'une quelconque des revendications précédentes, dans lequel la membrane (9) est fixée sur ladite surface environnante au moyen d'un adhésif (23).

8. Dispositif de cathéter urinaire à implantation externe selon l'une quelconque des revendications 1 à 6, dans lequel la membrane (9) est fixée sur ladite surface environnante au moyen d'un élément formant rivet (24).

9. Dispositif de cathéter urinaire à implantation externe selon l'une quelconque des revendications 1 à 6, dans lequel la membrane (9) est fixée sur ladite surface environnante par soudage.

10. Dispositif de cathéter urinaire à implantation externe selon l'une quelconque des revendications 1 à 9, dans lequel la membrane (9) est conçue pour permettre le passage d'urine à une pression supérieure à 80 mbar au-dessus de la pression ambiante.

11. Dispositif de cathéter urinaire à implantation externe selon l'une quelconque des revendications précédentes, dans lequel ledit orifice (8) est prévu dans le conduit d'évacuation (7) de l'élément de contact (1) et ledit écran (10) comprend un élément tubulaire (20) entourant le conduit d'évacuation (7) pour recouvrir ledit orifice (8) et la membrane (9) fixée sur ladite surface environnante.

12. Dispositif de cathéter urinaire à implantation externe selon les revendications 6 et 11, dans lequel ledit élément tubulaire (20) fait partie de l'élément formant support extérieur (12).

13. Dispositif de cathéter urinaire à implantation externe selon la revendication 12, dans lequel l'élément formant support extérieur (12) est immobilisé dans au moins le sens axial par rapport à l'élément intérieur (1) par l'intermédiaire d'un moyen de fixation.

14. Dispositif de cathéter urinaire à implantation externe selon la revendication 13, dans lequel l'élément formant support extérieur (12) est immobilisé dans le sens axial ainsi qu'en rotation par rapport à l'élément intérieur (1) par l'intermédiaire d'un moyen de fixation.

15. Dispositif de cathéter urinaire à implantation externe selon la revendication 13 ou 14, dans lequel ledit élément tubulaire (20) fait partie intégrante dudit élément formant support extérieur (12) et est raccordé par ledit moyen de fixation à la partie proximale du conduit d'évacuation (7) de l'élément intérieur (1).

16. Dispositif de cathéter urinaire à implantation externe selon la revendication 15, dans lequel ledit moyen de fixation se compose d'une ou de plusieurs saillies (31) ménagées dans la face intérieure de l'élément tubulaire (20) et, ou encore, la face extérieure du conduit d'évacuation (7).

17. Dispositif de cathéter urinaire à implantation externe selon la revendication 15, dans lequel ledit moyen de fixation se compose d'un dispositif à membrane (34) disposé dans une ouverture traversant ledit élément tubulaire (20) ainsi que ladite partie proximale du conduit d'évacuation (7).

18. Dispositif de cathéter urinaire à implantation externe selon l'une quelconque des revendications 13 à 15, dans lequel ledit moyen de fixation comprend une liaison de raccordement réalisée par soudage par un apport de chaleur ou aux ultrasons.

19. Dispositif de cathéter urinaire à implantation externe selon l'une quelconque des revendications précédentes, dans lequel ledit dispositif d'ouverture comprend au moins une ouverture (11, 14) ménagée dans ledit écran (10) en communication d'écoulement avec la surface extérieure de la membrane (9).

20. Dispositif de cathéter urinaire à implantation externe selon l'une quelconque des revendications précédentes, dans lequel la membrane (9) est soumise à l'action d'une dépression comparativement à ladite surface environnante de manière à assurer un dégagement par rapport audit écran (10) et ledit dispositif d'ouverture comprend au moins un sillon (18, 29, 30) formé par un creux dans la surface intérieure dudit écran (10) et, ou encore, ladite portion de surface environnante pour permettre une communication d'écoulement gazeux entre ledit dégagement et l'extérieur du dispositif de cathéter.

21. Procédé de fabrication d'un dispositif de cathéter urinaire à implantation externe pour remédier à l'incontinence urinaire masculine, qui comprend les étapes consistant à
prévoir un élément formant cathéter comprenant un élément de contact (1) agencé pour être placé, en position d'utilisation, en prise d'engagement avec au moins la partie extrême (3) d'un pénis (4), ledit élément de contact comprenant une portion d'extrémité distale (6),
réaliser une ouverture (5) implantée pour être positionnée sensiblement en face de l'orifice de l'urètre par ladite mise en prise d'engagement de l'élément de contact (1) avec ladite portion d'extrémité (3) et un conduit d'évacuation (7) raccordé à l'ouverture (5),
réaliser un orifice (8) dans la partie distale de l'élément de contact (1) ou dans le conduit d'évacuation (7),
fixer une membrane (9) sur la surface entourant ledit orifice (8), la membrane étant capable de manière sélective de laisser passer les gaz mais de retenir l'urine, **caractérisée en ce qu'**elle prévoit un écran de protection extérieur (10) pour recouvrir au moins de manière partielle la membrane et ménager au moins un dispositif d'ouverture (11, 14, 18, 29, 30) prévu pour permettre aux gaz d'être pratiquement librement en contact avec la surface extérieure de la membrane.

22. Procédé selon la revendication 21, dans lequel ladite ouverture (8) est ménagée dans le conduit d'évacuation (7) de l'élément de contact (1) et ledit écran (10) comprend un élément tubulaire (20) et est prévu pour entourer le conduit d'évacuation (7) de manière à recouvrir ladite ouverture (8) et la membrane (9) fixée sur ladite surface environnante.

23. Procédé selon la revendication 21 ou 22, comprenant en outre l'étape consistant à munir l'élément formant cathéter d'un élément de support extérieur (12) destiné à maintenir l'élément de contact (1) dans sa position d'utilisation en tant qu'élément intérieur entre le prépuce (2) et la gaine fibreuse (3) d'un pénis (4), ledit élément de support extérieur (12) se présentant sous la forme d'un segment d'extrémité distant de celui-ci, muni d'une ouverture pour le passage dudit conduit d'évacuation (7) et pouvant être disposé, dans sa position d'utilisation, dans une position axiale sensiblement fixe par rapport au conduit d'évacuation (7) pour être mise en prise d'engagement au niveau de sa circonférence avec la surface extérieure du prépuce (2).

24. Procédé selon les revendications 22 et 23, dans lequel ledit élément tubulaire (20) fait partie de l'élément de support extérieur (12).

25. Procédé selon la revendication 24, dans lequel l'élément de support extérieur (12) est immobilisé dans au moins la direction axiale par rapport à l'élément intérieur (1) par l'intermédiaire d'un moyen de fixation.

26. Procédé selon la revendication 25, dans lequel l'élément tubulaire (20) fait partie intégrante dudit élément de support extérieur (12) et ledit moyen de fixation est prévu pour raccorder ledit élément tubulaire (20) à la partie proximale du conduit d'évacuation (7) de l'élément intérieur (1).

27. Procédé selon la revendication 25 ou 26, dans lequel ledit moyen de fixation est réalisé par soudage par un apport de chaleur ou aux ultrasons.

28. Procédé selon l'une quelconque des revendications 21 à 27, dans lequel ledit élément formant cathéter est réalisé par un procédé comprenant les étapes consistant à
a) constituer une solution ou une émulsion de polymère liquide se composant d'un polymère et d'un solvant ou un diluant dans une quantité suffisante pour permettre un moulage par immersion,
b) constituer un moule comprenant une première partie pour réaliser par moulage ledit élément de contact, une deuxième partie pour constituer ladite ouverture dans la portion d'extrémité distale de l'élément de contact et une troisième partie pour réaliser par moulage ledit conduit d'évacuation, lesdites première, deuxième et troisième parties étant en l'occurrence réalisées solidaires,
c) immerger ledit moule dans ladite solution ou émulsion,
d) retirer le moule de la solution ou de l'émulsion,
e) laisser le solvant ou le diluant s'évaporer et
f) de manière facultative, répéter les étapes c), d) et e) jusqu'à ce que le dispositif ait atteint l'épaisseur désirée, et dans lequel, avant le processus d'immersion conformément à l'étape c) ou durant l'évaporation conformément à l'étape e), ladite membrane est placée au niveau de la partie du moule destinée à mouler la portion distale de l'élément de contact ou au niveau de la troisième partie du moule destinée à réaliser par moulage le conduit d'évacuation.

29. Procédé selon l'une quelconque des revendications 21 à 27, dans lequel ledit élément formant cathéter est réalisé par un procédé comprenant les étapes consistant à
constituer un moule se composant d'une matrice et d'un élément formant noyau définissant une empreinte pour moulage par injection de matière plastique, ladite empreinte se composant d'une première partie pour réaliser par moulage ledit élément de contact, une deuxième partie pour constituer ladite ouverture dans la portion d'extrémité distale de l'élément de contact et une troisième partie pour réaliser par moulage ledit conduit d'évacuation, lesdites première, deuxième et troisième parties étant en l'occurrence réalisées solidaires,
injecter une matière plastique liquide dans ladite empreinte de moule,
laisser la matière plastique liquide se solidifier et
retirer la pièce moulée du moule et dans lequel ladite membrane est placée au niveau de la partie de l'empreinte destinée à mouler la portion distale de l'élément de contact ou de la troisième partie de l'empreinte destinée à mouler le conduit d'évacuation.
